# EUROPEAN PATENT APPLICATION

(11) **EP 4 385 639 A1**
(43) Date of publication of application: **19.06.2024**
(21) Application number: 23216483.0
(22) Date of filing: 13.12.2023
(51) Int. Cl.: B21F 45/00, A61M 25/01, B21F 3/04

(54) **METHOD AND DEVICE FOR MANUFACTURING AN ELECTROMAGNETIC NAVIGATION SENSOR**

(30) Priority: 14.12.2022 US 202263432648 P; 14.11.2023 US 202318508909
(71) Applicant: Covidien LP, Mansfield, MA 02048 (US)
(72) Inventor: KNUTSON, Nathan J., Plymouth, 55441 (US)
(74) Representative: Maschio & Soames IP Ltd

(57) **Abstract**

A method and device for forming a sensor, the method including winding a desired length of wire from a first spool to a second spool, transferring the second spool to a spindle mounted in axial alignment with a substrate, securing the wire to a substrate, rotating the substrate about its axis such that the wire is drawn from the first spool to form a wound region, transferring the second spool to a twist former on which the first spool is mounted, and rotating the first spool and the second spool relative to each other to form a twist pair from the wire.

## Description

### CROSS-REFERENCE TO RELATED APPLICATION

This application claims priority to and the benefit of U.S. Provisional Application Serial No. 63/432,648, filed on December 14, 2022, the entire contents of which being incorporated by reference herein.

### BACKGROUND

### 1. Technical Field

The present disclosure relates to sensors and methods of manufacturing sensors for incorporation into tools and catheters for luminal navigation.

### 2. Discussion of Related Art

A common interventional procedure in the field of pulmonary medicine is bronchoscopy, in which a bronchoscope is inserted into the airways through the patient's nose or mouth. The structure of a bronchoscope generally includes a long, thin, flexible tube that typically contains three elements: an illumination assembly for illuminating the region distal to the bronchoscope's tip via an optical fiber connected to an external light source; an imaging assembly for delivering back a video image from the bronchoscope's distal tip; and a lumen or working channel through which instruments may be inserted, including, but not limited to, placement instruments (e.g., guide wires), diagnostic instruments (e.g., biopsy tools) and therapeutic instruments (e.g., treatment catheters or laser, cryogenic, radio frequency, or microwave tissue treatment probes).

During some procedures (e.g., microwave ablation and biopsy), a catheter or another tool may be inserted through a working channel of the bronchoscope to enable navigation and therapy at sites that are too remote, or have luminal diameters too small, for navigation of the bronchoscope. The catheter or tool may include a sensor at its distal end to assist in guiding the catheter to targeted tissue. Where a catheter is being used, and once positioned adjacent targeted tissue, an instrument may be inserted through the extended working channel of the catheter to perform a procedure on the targeted tissue (e.g., perform a biopsy or ablation of the targeted tissue).

In some current offerings, the sensor is fabricated using a plurality of discreet wires that require a metal bonding connection (e.g., soldering). Since the distal end of the catheter or tool is subjected to bending forces during use, the sensor and its connections experience strain that can result in failure.

Accordingly, there is a need for catheters with a locatable sensor having a longer useful life.

### SUMMARY

One aspect of the disclosure is directed to a method of forming a sensor. The method also includes securing a first spool in a twist former. The method also includes securing a second spool on a spindle mounted in axial alignment with a substrate; securing the wire to a substrate, rotating the substrate about its axis such that the wire is drawn from the first spool to form a wound region, transferring the second spool to the twist former on which the first spool is mounted, rotating the first spool and the second spool relative to each other to form a twist pair from the wire. Other embodiments of this aspect include corresponding computer systems, apparatus, and computer programs recorded on one or more computer storage devices, each configured to perform the actions of the methods and systems described herein.

Implementations of this aspect of the disclosure may include one or more of the following features. The method where the substrate is removable from the wound region of the sensor. The method further including binding the wound region such that wraps of the wire around the substrate bond to each other. The method, further including mounting the second spool in spool pre-winder, and winding the wire from the first spool to the second spool mounted in the spool pre-winder. The method further including passing the wire through a wire guide to secure the wire to the substrate. The method further including threading the wire on the second spool through the wire guide when transferring the second spool to the twist former. The method further including tensioning the wire such that the wound region is tightly wound, and that the wire does not break. Implementations of the described techniques may include hardware, a method or process, or computer software on a computer-accessible medium, including software, firmware, hardware, or a combination of them installed on the system that in operation causes or cause the system to perform the actions. One or more computer programs can be configured to perform particular operations or actions by virtue of including instructions that, when executed by data processing apparatus, cause the apparatus to perform the actions.

One aspect of the disclosure is directed to a sensor winding device including a pair of headstocks aligned along a first axis, each headstock having a chuck for receiving and securing a substrate therebetween; a twist former including two spindles each configured to receive either a first spool or a second spool and a shaft, the two spindles configured to rotate by rotation of the shaft; a spool support mounted on one of the chucks and configured to receive the second spool; and a wire housed partially on the first spool and partially on the second spool, where with the second spool secured on the spool support, rotation of the chucks and therewith the substrate, a wound portion of a sensor is formed, and where with both the first spool and second spool mounted in the two spindles rotation of the shaft forms a twist pair portion of the sensor. Other embodiments of this aspect include corresponding computer systems, apparatus, and computer programs recorded on one or more computer storage devices, each configured to perform the actions of the methods and systems described herein.

Implementations of this aspect of the disclosure may include one or more of the following features. The sensor winding device further including a pair of arms extending from the shaft of the twist former. The sensor winding device further including a tensioner mounted on the arms and configured to ensure proper tension is applied to prevent breakage. The sensor winding device further including a wire guide. The pair of headstocks are mounted on the carriage and movement of carriage relative to the wire guide ensures placement of the wire to form the wound portion. The twist former is mounted on the post. The pre-winder is configured to draw wire from the first spool. The second spool is movable from the pre-winder to the spool support and the spindles of the twist former. Implementations of the described techniques may include hardware, a method or process, or computer software on a computer-accessible medium, including software, firmware, hardware, or a combination of them installed on the system that in operation causes or cause the system to perform the actions. One or more computer programs can be configured to perform particular operations or actions by virtue of including instructions that, when executed by data processing apparatus, cause the apparatus to perform the actions.

One aspect of the disclosure is directed to a method of forming a sensor. The method also includes winding a desired length of wire on a second spool with a pre-winder, the pre-winder drawing wire from a first spool mounted on a spindle associated with a twist former; mounting a substrate between a pair of chucks; transferring the second spool to a spindle mounted in axial alignment with a substrate; securing the wire to the substrate, such that the wire extends from the first spool through a wire guide to the substrate and to the second spool; rotating the pair of chucks about an axis such that the wire is drawn from the first spool to form a wound region on the substrate; driving a carriage to which the chucks are operably connected, such that axial movement of the carriage forces the wire against the wire guide to form a wound portion of the sensor; transferring the second spool to the twist former, where a portion of the wire extending from the second spool is led between two posts of the wire guide and parallel the portion of the wire extending from the first spool; rotating the first spool and the second spool relative to each other to form a twist pair from the wire; and driving a second carriage until a twisted pair of desired length is achieved. Other embodiments of this aspect include corresponding computer systems, apparatus, and computer programs recorded on one or more computer storage devices, each configured to perform the actions of the methods and systems described herein.

Implementations of this aspect of the disclosure may include one or more of the following features. The method where the carriage is driven in a first direction for a first length and a second direction for the first length. The desired length is half the length of the wire required for a desired length of sensor. The method further including tensioning the wire to prevent breakage and ensure smooth formation of the wound region. The method further including binding the wire in the wound region and removing the substrate. Implementations of the described techniques may include hardware, a method or process, or computer software on a computer-accessible medium, including software, firmware, hardware, or a combination of them installed on the system that in operation causes or cause the system to perform the actions. One or more computer programs can be configured to perform particular operations or actions by virtue of including instructions that, when executed by data processing apparatus, cause the apparatus to perform the actions.

### BRIEF DESCRIPTION OF THE DRAWINGS

Fig. 1 is a perspective view of a sensor and twisted pair formed in accordance with the disclosure;

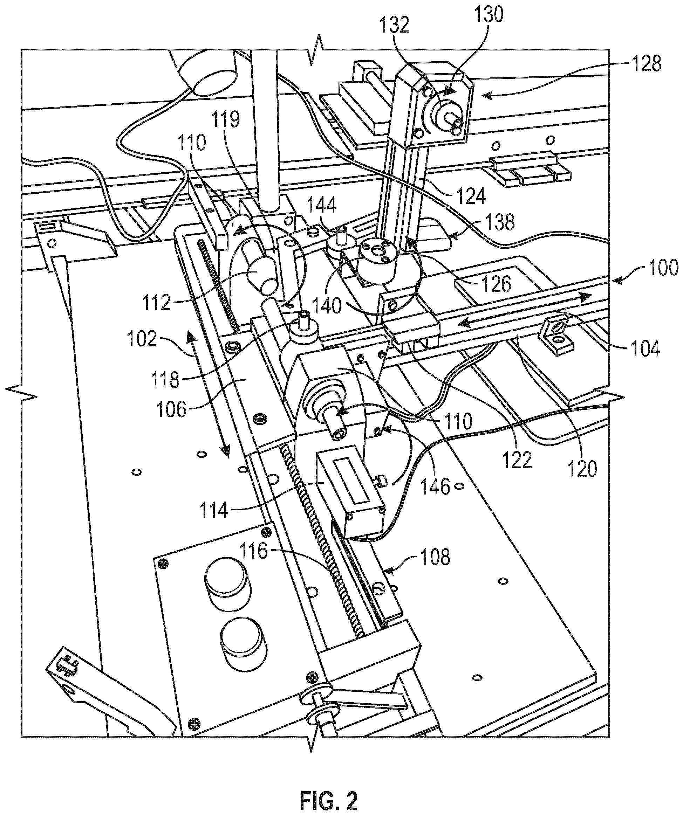

Fig. 2 is a top perspective view of a sensor forming device in accordance with the disclosure;
Fig. 3 is a front view of the sensor forming device of Fig. 2; and

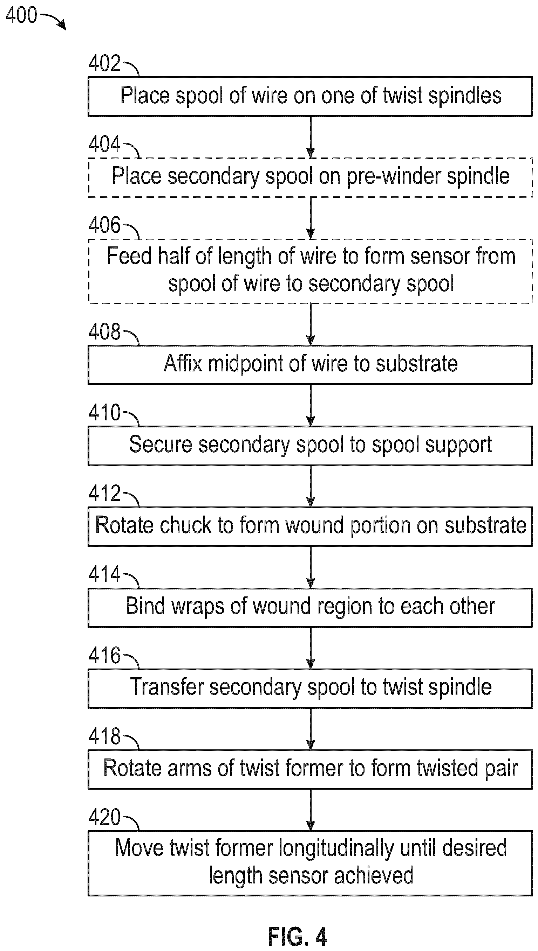

Fig. 4 is a flow chart detailing a method of manufacturing the sensor of Fig. 1.

### DETAILED DESCRIPTION

This disclosure is directed to devices and methods of forming sensors, particularly electromagnetic sensors. The sensors are configured for placement near a distal end of a tool or catheter that is employed for navigation within a luminal network. In particular, the tool or catheter are configured to navigate the airways of the lungs.

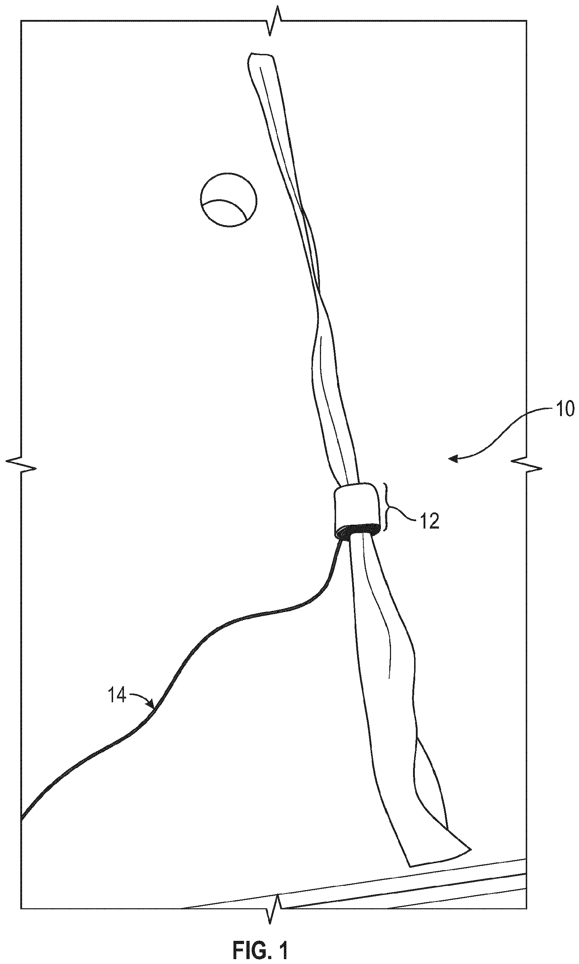

As shown in Fig. 1, the sensor 10 is formed of a wound region 12, formed of a plurality of wraps of a conductive wire. On a proximal end of the sensor 10, the windings terminate and the wires 14 extend away from the wound region 12. To eliminate interference and notice in electrical systems associated with the catheter or tool on which the sensor 10 is to be placed the wires 14 are formed into a twisted pair. A proximal end (not shown) of the twisted pair can be connected to terminals that are connectable to a navigation system. The navigation system includes an electromagnetic field generator. By placing the sensor 10 in the electromagnetic field, a current is generated in the wound region 12 and transmitted to the navigation system via the twisted pair of wires 14.

As noted above, historically, the wound portion 12 would be formed in one step, and the twisted pair of wires 14 formed in a second. These two components then require a soldering, or other mechanical connection step to electrically connect the components. But a catheter or tool for luminal navigation in the lungs is required to bend and twist for navigation and also survive the movement stresses caused by respiration.

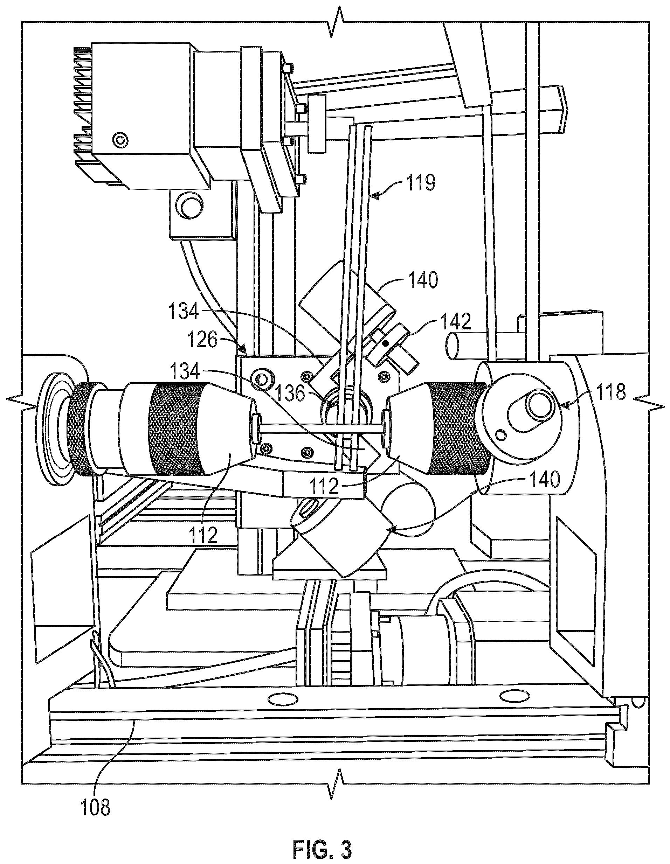

To form a sensor 10 in accordance with the disclosure formed without solder or other mechanical joining of the wound portion 12 and twisted pair 14, a winding machine 100 is employed. The winding machine 100 defines a first axis 102 and a perpendicular second axis 104. On the first axis is a carriage 106 which rides on first rail 108. The carriage 106 supports two head stocks 110, each headstock 110 including a chuck 112 for grasping a mandrel or another substrate or structure that spans a gap between the two chucks 112 (See Fig. 3). Either or both chucks 112 may be driven by a motor 114. In one implementation of the winding machine 100, both chucks 112 are synchronously driven by motors 114, however in other implementations one of the chucks 112 is merely passive and not itself motor driven but rather is rotatably coupled to the driven headstock 110 and chuck 112 through the substrate . Still further, if the substrate is sufficiently rigid, the second headstock 110 and chuck 112 can be entirely eliminated and only a single headstock 110 and chuck 112 is employed..

A separate motor (not shown) is connected to linear screw 116, which interfaces with and drives the carriage 106 along the first axis 102 defined by the first rail 108. Mounted on one of the chucks 112, such that it rotates with the chuck 112 is a spool support 118 which is configured to receive a spool of wire, the use of which will be described in greater detail below. A wire guide 119 is configured to have the wire pass therethrough, the wire guide helps ensure that each successive coil of wire butts against the prior coil to form a cohesive wound portion 112. In one embodiment the wire guide 119 remains stationary while the headstocks 110 rotate and are advanced along the first axis 102 by the linear screw 116. Alternatively, the wire guide 119 may be mounted on the carriage 106, such that the linear screw 116 moves the wire guide 119, while the chucks 112 rotate but are otherwise fixed in location. Still further both the wire guide 119 and the headstocks 110 may be motor driven and move either in concert or independently.

In accordance with aspects of the disclosure the substrate that spans the gap between the two chucks 112 can be formed of a variety of materials. In one aspect the substrate is a material that has rigidity when under tension between the two chucks 112, but once the tension is released looses its rigidity and can be easily removed from the wound portion 12 of the sensor 10. Alternatively, the substrate may be a rigid reusable mandrel, such as a rod of steel or other metal, that can be removed from the wound portion 12 of the sensor 10 for incorporation into a catheter or tool. Still further, the substrate may be a polymeric or metal material and intended to form a component of the catheter or tool. For example, if a polymeric material, the substrate may be formed of a similar heat flowable material as other components of the catheter or tool and joined with these materials through the catheter or tool manufacturing process. Similarly, where formed of a metal or other material resistant to piercing, the senor 10, on the substrate can be joined with other portions of the catheter, where when a tool is inserted into the catheter, the substrate protects the sensor 10, and particularly the wound portion 12 from being pierced by the tool as it navigates the internal lumen of the catheter. Materials of which the substrate may formed include steel, Nitinol, titanium, polytetrafluoroethylene (PTFE), Arnitel^{®}, polyether ether ketone (PEEK), and others.

In some aspects of the disclosure, though described as axially aligned, the headstocks 110 may be as much as 20 to 30 degrees out of axial alignment. Where the substrate is flexible or very thin diameter (e.g., 0.005-0.010 in. diameter), even with axially aligned headstocks 110 rotation of the substrate results in radial bowing of the substrate as it rotates between the headstocks 110. This bowing can be countered locally by allowing or forcing the substrate to contact the wire guide 119. The wire guide 119 may be treated with molybdenum or PTFE to act as a bearing surface. Rotation of the substrate and tension applied to the substrate by the wire and tensioner 140 forces the substrate against the wire guide 119, which then acts as a bearing, which produces a portion of the substrate to be substantially straight near the wire guide 119, thus allowing the formation of a wound portion 12 on a straight portion of the substrate, despite the outward bowing caused by the rotation of the substrate. By placing the headstocks 110 out of alignment, the formation of the flat portion can be adjusted as needed.

Though, generally described herein as having coils of the would portion be formed with the wire orthogonal to the substrate other angular orientations are contemplated herein. For example, the coils may be formed at any angle between 20 and 70 degrees askew relative to the longitudinal axis of the substrate.

The second axis 104 of the winding machine 100 is defined by a second rail 120. The second rail 120 supports a second carriage 122 on which is mounted a post 124. The post 124 supports a twist former 126 and a spool pre-winder 128. The spool pre-winder 128 includes a motor 130 and a spindle 132 connected thereto and configured to receive a secondary spool (not shown), as described below. The twist former 126 includes a twisting assembly including two arms 134 extending from a shaft 136. A motor 138 connects to the two arms 134 via the shaft 136. Proximate an end of each arm 134 opposite the shaft 136 is mounted of a tensioner 140 which is operably connected to a twist spindle 142. Each twist spindle 142 is configured to receive a spool 144, as described in greater detail below. Motor 146 moves the second carriage 122 and the post 124 along the axis 104 defined by rail 120 as the twist former rotates forming a twisted pair 14 of wires.

A method 400 can be employed to form a sensor 10 in accordance with the disclosure. In accordance with method 400 a spool of wire 144 is placed in one of the twist spindles 142 at step 402. At step 404 a secondary spool (not shown but similar to 144) is placed in the spindle 132 of the spool pre-winder 128. Because the diameter and length of the wound region 12 are known, as is a desired length of the twisted pair of wires 14, the overall length of the wire needed to form the sensor 10 is known. At step 406 approximately half of the length of wire to form sensor 10 is feed from the spool of wire 144 and to the secondary spool supported by spindle 132. This can be accomplished using motor 130 to rotate the empty spool while a tensioner 140 connected to twist spindle 142 on which the spool of wire 144 is secured. The tensioner ensures that proper tension is maintained on both the twist spindle 142 and the spindle 132 to prevent breakage of the wire by over tensioning or the loose winding of the wire on the empty spool as it is driven by the motor 130. Those of skill in the art will recognize that steps 404 and 406 are optional, and the secondary spool may be pre-loaded with wire such that no pre-winding is required.

Once approximately half the length of wire needed for formation of the sensor 10 is transferred from the spool 144 to the secondary spool, a portion of the wire is threaded through the wire guide 119 and affixed to a substrate supported by the two chucks 112 and spanning the gap therebetween at step 408. The wire may be affixed to the substrate with a clip, tape, skyve, or a slot. Once the wire is affixed to the substrate, which may be a mandrel of appropriate diameter, a thin piece of tubing, a catheter liner (e.g., PTFE) or another structure capable of supporting the wire and rotating on its axis between the chucks 112.

With the wire affixed to the substrate at approximately its midpoint, the secondary spool is moved from the spindle 132 to spool support 118 on the head piece 110 at step 410. Those of skill in the art will recognize that the order of steps 408 and 410 may be reversed without departing from the scope of the disclosure. The chucks 110 rotate at step 412 to draw wire from the spool 144 secured in the twist spindle 142, again the tensioner 140 ensures that the wire is always under tension as it is being wound around the substrate, but similarly not over tensioned. Following, formation of the wound region 12, which may be formed for example by a first wrapping of the wire on the catheter in a first direction, and ten a second wrapping over the first wrapping, the wound region 12, and the portions of the wire therein, is bonded to itself to form a cohesive wound region 12 at step 414. This bonding may be by a heat bond, a solvent bond, varnish, or a wicking adhesive, as well as others. Forming the wound portion 12 by wrapping the wire in a first direction and then by forming a second layer by wrapping in a second direction the wire extending from the wound portion 12 is proximate the portion of the wire leading to the secondary spool.

Those of skill in the art will appreciate that though described above in connection with a two-layer wound portion 14, the disclosure is not so limited. Naturally even number layers will result in the portions of the wire leading to the secondary spool and to the spool 144 on the twist spindle 142 are near located near one another and allowing for easy formation of the twisted pair (described below). However, odd numbers of layers may also be employed in the wound portion 14. To achieve an odd number of layers either an under layer (e.g., under the wound portion) or an over layer (over the wound portion) of wire is employed to bring the bring the wire into position for forming of the twisted pair, as described above. The overlayer or underlayer, which is a straight section of wire generally perpendicular to the coils of the would portion, can be accommodated in a final construction of a catheter or tool, as the polymeric materials of the catheter or tool, when reflowed, conform to this slight incongruity of shape.

At step 416, the secondary spool is transferred from the spool support 118, threaded through the wire guide to one of the twist spindles 142 such that there are two wires extending from the wound region 12, through the wire guide 119, and connecting to spool 144 and the secondary spool each of which is secured to a twist spindle 142. At step 418, the motor 138 rotates the two arms 134 via shaft 136, causing the wires to twist and form a twisted pair. At step 420 the carriage drive motor 146 operably connected to the second carriage 122 draws the twist former 128 along the second axis 104 defined by the second rail and away from the first axis 102 until a twisted pair of sufficient length has been formed, or until the spool 144 or secondary spool are exhausted of wire at which the procedure ends.

The sensor 10 so formed can be removed from the substrate and made ready for incorporating into a tool or catheter for luminal navigation. Moreover, this sensor 10 is formed from a single length of wire with no transitions or mechanical connections. In this manner, the sensor 10 need not be formed on the catheter as part of the catheter manufacturing process, but rather can be a component added to the buildup of the catheter layers. One advantage of this is that the twisted pair need not be wrapped around the catheter, but rather can be inserted into longitudinal spacing in the layers of the catheter, reducing the amount of wire needed, and the likelihood of breaking of the wire during manufacturing.

While the first axis 102 and second axis 104 are described herein as being perpendicular to one another they need not be so configured and can be arranged at any angle through the use of a gripper or a wire guide similar to wire guide 119 enabling the change of relative angle of the axes.

The dual headstocks 110, with synchronously driven motors 114, enable the formation of sensors 10 where the wound region 12 can have a core diameter as small as 0.0002 inches. These small diameter wound regions 12 require very small diameter substrates or mandrels, and the dual headstocks 110 limit the radial deflection of the substrate or mandrel as the wound region 12 is formed. Where a larger diameter wound region 12 is desired, or where the substrate or mandrel is formed of a stiffer material, only one of the headstocks 110, and the chuck 112 need be driven by a motor 114.

The tensioners 140 can take a number of configurations including passive magnetic tensioners, electromagnetic, variable electromagnetic using slip ring unitions or variable brakes as well as other configurations known to those of skill in the art.
The invention may be described by reference to the following numbered paragraphs:-
1. A method of forming a sensor comprising:
   securing a first spool in a twist former;
   securing a second spool on a spindle mounted in axial alignment with a substrate;
   securing the wire to the substrate;
   rotating the substrate about its axis such that the wire is drawn from the first spool to form a wound region;
   transferring the second spool to the twist former on which the first spool is secured;
   rotating the first spool and the second spool relative to each other to form a twist pair from the wire.
2. The method of paragraph 1, wherein the substrate is removable from the wound region of the sensor.
3. The method of paragraph 1, further comprising binding the wound region such that windings of the wire around the substrate bond to each other.
4. The method of paragraph 1, further comprising mounting the second spool in a spool pre-winder, and winding the wire from the first spool to the second spool mounted in the spool pre-winder.
5. The method of paragraph 1, further comprising passing the wire through a wire guide to secure the wire to the substrate.
6. The method of paragraph 1, further comprising threading the wire on the second spool through the wire guide when transferring the second spool to the twist former.
7. The method of paragraph 1, further comprising tensioning the wire such that the wound region is tightly wound.
8. A sensor winding device comprising:
   a pair of headstocks aligned along a first axis, each headstock having a chuck for receiving and securing a substrate therebetween;
   a twist former including two spindles each configured to receive either a first spool or a second spool and a shaft, the two spindles configured to rotate by rotation of the shaft;
   a spool support mounted on one of the chucks and configured to receive the second spool; and
   a wire housed partially on the first spool and partially on the second spool, wherein with the second spool secured on the spool support, rotation of the chucks forms a wound portion of a sensor, and wherein with both the first spool and second spool mounted in the two spindles, rotation of the shaft forms a twist pair portion of the sensor.
9. The sensor winding device of paragraph 8, further comprising a pair of arms extending from the shaft of the twist former.
10. The sensor winding device of paragraph 9, further comprising a tensioner mounted on the arms and configured to cause tension to be applied to the wire to prevent breakage.
11. The sensor winding device of paragraph 10, further comprising a wire guide.
12. The sensor winding device of paragraph 11, further comprising a first carriage and a linear screw configured to drive the carriage along a first axis, wherein the pair of headstocks are mounted on the carriage and movement of the carriage relative to the wire guide cause placement of the wire to form the wound portion.
13. The sensor winding device of paragraph 12, further comprising a second carriage configured to move along a second axis and a post mounted on the second carriage, wherein the twist former is mounted on the post.
14. The sensor winding device according to paragraph 13, further comprising a pre-winder, configured to receive the second spool, wherein the pre-winder is configured to draw wire from the first spool.
15. The sensor winding device of paragraph 14, wherein the second spool is movable from the pre-winder to the spool support and the spindles of the twist former.
16. A method of forming a sensor comprising:
   winding a length of wire on a second spool with a pre-winder, the pre-winder drawing wire from a first spool mounted on a spindle associated with a twist former;
   mounting a substrate between a pair of chucks;
   transferring the second spool to a spindle mounted in axial alignment with a substrate;
   securing the wire to the substrate, such that the wire extends from the first spool through a wire guide to the substrate and to the second spool;
   rotating the pair of chucks about an axis such that the wire is drawn from the first spool to form a wound region on the substrate;
   driving a carriage to which the chucks are operably connected, such that axial movement of the carriage forces the wire against the wire guide to form a wound portion of the sensor;
   transferring the second spool to the twist former, wherein a portion of the wire extending from the second spool is led between two posts of the wire guide and parallel to the portion of the wire extending from the first spool;
   rotating the first spool and the second spool relative to each other to form a twist pair from the wire; and
   driving a second carriage until a length of twisted pair is achieved.
17. The method according to paragraph 16, wherein the carriage is driven in a first direction for a first distance and a second direction for the first distance.
18. The method according to paragraph 16, wherein the length of wire wound on the second spool is half the length of the wire required for the formation of the sensor.
19. The method according to paragraph 16, further comprising tensioning the wire to prevent breakage and cause smooth formation of the wound region.
20. The method according to paragraph 16, further comprising binding the wire in the wound region and removing the substrate.

## Claims

1. A method of forming a sensor comprising:
securing a first spool in a twist former;
securing a second spool on a spindle mounted in axial alignment with a substrate;
securing the wire to the substrate;
rotating the substrate about its axis such that the wire is drawn from the first spool to form a wound region;
transferring the second spool to the twist former on which the first spool is secured;
rotating the first spool and the second spool relative to each other to form a twist pair from the wire.

2. The method of claim 1, wherein the substrate is removable from the wound region of the sensor.

3. The method of claim 1 or claim 2, further comprising binding the wound region such that windings of the wire around the substrate bond to each other.

4. The method of any preceding claim, further comprising mounting the second spool in a spool pre-winder, and winding the wire from the first spool to the second spool mounted in the spool pre-winder.

5. The method of any preceding claim, further comprising passing the wire through a wire guide to secure the wire to the substrate and /or further comprising threading the wire on the second spool through the wire guide when transferring the second spool to the twist former.

6. The method of any preceding claim, further comprising tensioning the wire such that the wound region is tightly wound.

7. A sensor winding device comprising:
a pair of headstocks aligned along a first axis, each headstock having a chuck for receiving and securing a substrate therebetween;
a twist former including two spindles each configured to receive either a first spool or a second spool and a shaft, the two spindles configured to rotate by rotation of the shaft;
a spool support mounted on one of the chucks and configured to receive the second spool; and
a wire housed partially on the first spool and partially on the second spool, wherein with the second spool secured on the spool support, rotation of the chucks forms a wound portion of a sensor, and wherein with both the first spool and second spool mounted in the two spindles, rotation of the shaft forms a twist pair portion of the sensor.

8. The sensor winding device of claim 7, further comprising a pair of arms extending from the shaft of the twist former; preferably further comprising a tensioner mounted on the arms and configured to cause tension to be applied to the wire to prevent breakage.

9. The sensor winding device of claim 8, further comprising a wire guide.

10. The sensor winding device of claim 9, further comprising a first carriage and a linear screw configured to drive the carriage along a first axis, wherein the pair of headstocks are mounted on the carriage and movement of the carriage relative to the wire guide cause placement of the wire to form the wound portion; preferably further comprising a second carriage configured to move along a second axis and a post mounted on the second carriage, wherein the twist former is mounted on the post.

11. The sensor winding device according to claim 10, further comprising a pre-winder, configured to receive the second spool, wherein the pre-winder is configured to draw wire from the first spool; preferably wherein the second spool is movable from the pre-winder to the spool support and the spindles of the twist former.

12. A method of forming a sensor comprising:
winding a length of wire on a second spool with a pre-winder, the pre-winder drawing wire from a first spool mounted on a spindle associated with a twist former;
mounting a substrate between a pair of chucks;
transferring the second spool to a spindle mounted in axial alignment with a substrate;
securing the wire to the substrate, such that the wire extends from the first spool through a wire guide to the substrate and to the second spool;
rotating the pair of chucks about an axis such that the wire is drawn from the first spool to form a wound region on the substrate;
driving a carriage to which the chucks are operably connected, such that axial movement of the carriage forces the wire against the wire guide to form a wound portion of the sensor;
transferring the second spool to the twist former, wherein a portion of the wire extending from the second spool is led between two posts of the wire guide and parallel to the portion of the wire extending from the first spool;
rotating the first spool and the second spool relative to each other to form a twist pair from the wire; and
driving a second carriage until a length of twisted pair is achieved.

13. The method according to claim 12, wherein the carriage is driven in a first direction for a first distance and a second direction for the first distance and/or wherein the length of wire wound on the second spool is half the length of the wire required for the formation of the sensor.

14. The method according to claim 12 or claim 13, further comprising tensioning the wire to prevent breakage and cause smooth formation of the wound region.

15. The method according to claim 14, further comprising binding the wire in the wound region and removing the substrate.
